# EUROPEAN PATENT APPLICATION

(11) **EP 4 112 613 A1**
(43) Date of publication of application: **04.01.2023**
(21) Application number: 21760837.1
(22) Date of filing: 25.02.2021
(51) Int. Cl.: C07D 307/93, C07D 405/04, C07D 405/10, C07D 409/10, C09K 11/06, H01L 51/50

(54) **ARYLAMINE COMPOUND AND ELECTRONIC APPARATUS USING SAME**

(30) Priority: 26.02.2020 JP 2020029982
(71) Applicant: Hodogaya Chemical Co., Ltd., Tokyo, 105-0021 (JP)
(72) Inventor: KASE, Kouki, Tokyo 104-0028 (JP); KO Sang-Won, Tokyo 104-0028 (JP); Ryu Jung-Ho, Tokyo 104-0028 (JP); Lee Bong-Hyang, Tokyo 104-0028 (JP); IZUMIDA, Junichi, Tokyo 104-0028 (JP)
(74) Representative: Forstmeyer, Dietmar
(86) International application number: PCT/JP2021/007162
(87) International publication number: WO 2021/172452

(57) **Abstract**

An object of the present invention is to provide a material for a highly efficient and highly durable organic EL device, and specifically, an organic compound with excellent properties including excellent hole-injecting/transporting performance, electron-blocking capability, and high stability in the form of a thin film. Another object of the present invention is to provide a highly efficient and highly durable organic EL device by using this compound. The present invention is directed to an arylamine compound having a fluorenyl skeleton-containing heterocycle structure, with excellent heat resistance and good hole-transporting capability. An organic EL device including this compound in its hole-transporting layer, electron-blocking layer, light-emitting layer, or hole-injecting layer exhibited good device characteristics.

## Description

### Technical Field

The present invention relates to a compound suitable for organic electroluminescence devices (hereinafter referred to simply as "organic EL devices"), which are self-light emitting devices favorably used in various display apparatus, and the organic EL device, and more particularly relates to an arylamine compound and an organic EL device including the compound.

### Background Art

Since organic EL devices are self-emissive devices, they have larger brightness and better viewability than devices including liquid crystals, and can provide a clearer display. For these reasons, active studies have been carried out on organic EL devices.

In 1987, C. W. Tang et al. of Eastman Kodak Company developed a device having a layered structure in which various functions were assigned to different materials, and thus made a practical organic EL device including organic materials. They made an organic EL device having a layered structure including a layer of a fluorescent substance capable of transporting electrons and a layer of an organic substance capable of transporting holes, and injected both charges into the layer of the fluorescent substance to thereby cause the layer to emit light, and the organic EL device thus achieved a luminance as high as 1,000 cd/m² or more at a voltage of 10 V or less (see Patent Literatures 1 and 2, for example).

Organic EL devices have been heretofore much improved to put them to practical use. Electroluminescent devices have been suggested in which an anode, a hole-injecting layer, a hole-transporting layer, a light-emitting layer, an electron-transporting layer, an electron-injecting layer, and a cathode are sequentially provided on a substrate to subdivide various functions in the multi-layered structure even further, and such electroluminescent devices successfully have high efficiency and durability (see Non-Patent Literature 1, for example).

To further increase luminous efficacy, attempts have been made to utilize triplet excitons, and the utilization of phosphorescent compounds has been investigated (see Non-Patent Literature 2, for example). Moreover, devices that utilize light emission by thermally activated delayed fluorescence (TADF) have also been developed. In 2011, Adachi et al. from Kyushu University achieved a result of an external quantum efficiency of 5.3% by a device including a thermally activated delayed fluorescence material (see Non-Patent Literature 3, for example).

The light-emitting layer can also be prepared by doping a charge-transporting compound, generally called a host material, with a fluorescent compound, a phosphorescent light emitting compound, or a material that radiates delayed fluorescence. As stated in the non-patent literature above, the selection of the organic materials in an organic EL device greatly affects the characteristics of that device, such as efficiency and durability (see Non-Patent Literature 2, for example).

In an organic EL device, the charges injected from both electrodes recombine in the light-emitting layer, thereby producing light emission, and how efficiently the both charges, i.e., the holes and the electrons, are transported to the light-emitting layer is of importance. Thus, the device needs to have excellent carrier balance. Accordingly, the probability of recombination of holes and electrons in the light-emitting layer can be increased by using a material that improves hole injectability, that is, the ability to supply holes injected from the anode to the light-emitting layer and improves electron block ability, that is, the ability to block electrons injected from the cathode, and furthermore, higher luminous efficacy can be achieved by confining excitons generated in the light-emitting layer. For these purposes, the functions of the hole-transporting material are important, and there is a demand for a hole-transporting material having high hole injectability, high hole mobility, high electron block ability, and high durability against electrons.

Moreover, heat resistance and amorphousness of the materials are also important for lifespan of the device. A material with low heat resistance thermally decomposes, due to heat generated during the operation of the device, even at a low temperature, and thus the material deteriorates. A film made of a material with low amorphousness causes crystallization thereof even in a short period of time to result in deterioration of the device. Thus, the materials to be used are required to have high heat resistance and good amorphousness.

N,N'-diphenyl-N,N'-di(α-naphthyl)benzidine (NPD) and various aromatic amine derivatives are known as hole-transporting materials that have been heretofore used for organic EL devices (see Patent Literatures 1 and 2, for example). NPD has good hole-transporting capability, but has a glass transition point (Tg), which is a measure of heat resistance, as low as 96°C. Such a glass transition point cause a deterioration of the device characteristics due to crystallization of NPD under high-temperature conditions (see Non-Patent Literature 4, for example).

Moreover, although the aromatic amine derivatives disclosed in Patent Literatures 1 and 2 include compounds having an excellent hole mobility of 10⁻³ cm²/Vs or higher, the electron-blocking capability thereof is insufficient. Thus, when using such a compound, some electrons pass through the light-emitting layer, and unfortunately, no increase in luminous efficacy can be expected. Accordingly, in order to further increase the efficacy, materials that have higher electron-blocking capability, higher stability in the form of a thin film, and higher heat resistance are needed. Furthermore, although aromatic amine derivatives with high durability have been reported (see Patent Literature 3, for example), these aromatic amine derivatives are used as charge-transporting materials for a photoconductor for electrophotography, and there are no precedents of application to an organic EL device.

With a view to addressing these issues, arylamine compounds having a substituted carbazole structure or a fluorenyl skeleton-containing heterocycle structure have been suggested as compounds improved in properties such as heat resistance and hole injectability (see Patent Literatures 4 and 5, for example). However, although devices including such a compound in a hole-injecting layer or a hole-transporting layer have improved heat resistance and luminous efficacy, the performance thereof are still insufficient. Therefore, there is demand for a further decrease in driving voltage and a further increase in luminous efficacy.

### Citation List

### Patent Literatures

Patent Literature 1: US 5792557
Patent Literature 2: US 5639914
Patent Literature 3: US 7759030
Patent Literature 4: US 8021764
Patent Literature 5: KR 2018-0082124
Patent Literature 6: EP 2684932

### Non-Patent Literatures

Non-Patent Literature 1: Proceedings of the 9th Meeting of the Japan Society of Applied Physics, pp. 55-61 (2001)
Non-Patent Literature 2: Proceedings of the 9th Meeting of the Japan Society of Applied Physics, pp. 23-31 (2001)
Non-Patent Literature 3: Appl. Phys. Let., 98, 083302 (2011)
Non-Patent Literature 4: Proceedings of the 3rd Meeting of the Japan OLED Forum, pp. 13-14 (2006)

### Summary of Invention

An object of the present invention is to provide a material for a highly efficient and highly durable organic EL device, and specifically, a material for an organic EL device with (1) excellent hole-injecting/transporting performance, (2) electron-blocking capability, (3) high stability in the form of a thin film, and (4) excellent durability.

Another object of the present invention is to provide, by using the material of the present invention, an organic EL device with (1) high luminous efficacy and high power efficiency, (2) a low voltage for the start of light emission and a low actual driving voltage, and (3) a long lifespan.

To achieve the above-described objects, the inventors of the present invention have focused on the fact that an arylamine compound having a fluorenyl skeleton-containing heterocycle structure is excellent in terms of hole-injecting/transporting performance, and stability and durability in the form of a thin film. Conventionally, an arylamine compound in which a nitrogen atom is directly combined with the heterocycle group has been developed as a hole-transporting material; however, if an arylene group is provided between the heterocycle group and the nitrogen atom, a material having a wide energy gap and a material having a high heat resistance can be developed, which significantly improves the material properties. In organic EL devices as well, electrons from the cathode side are blocked due to the wide energy gap, and electron outflow is thus prevented to confine them in the light-emitting layer, which contributes to an improvement in the luminous efficacy and the power efficiency. Furthermore, the improvement in the luminous efficacy suppresses power consumption, and thus the load in the devices can be reduced. Also, the improvement in the heat resistance of the material improves the stability in forming a thin film, which enables a lifespan longer than that of conventional devices (see Patent Literature 5). Thus, the present invention has been completed.
1) Specifically, the present invention is directed to an arylamine compound having a fluorenyl skeleton-containing heterocycle structure represented by a general formula (A) below. 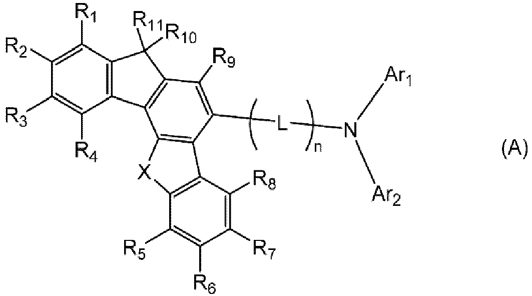
   where R₁ to R₁₁ is the same or different, and each represent a hydrogen atom, a deuterium atom, a fluorine atom, a chlorine atom, a cyano group, a nitro group, a linear or branched alkyl group having 1 to 6 carbon atoms and optionally having a substituent, a cycloalkyl group having 5 to 10 carbon atoms and optionally having a substituent, a linear or branched alkenyl group having 2 to 6 carbon atoms and optionally having a substituent, a linear or branched alkyloxy group having 1 to 6 carbon atoms and optionally having a substituent, a cycloalkyloxy group having 5 to 10 carbon atoms and optionally having a substituent, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group;
   L represents a divalent group of a substituted or unsubstituted aromatic hydrocarbon group, a substituted or unsubstituted aromatic heterocyclic group, or a substituted or unsubstituted fused polycyclic aromatic group;
   n is 1 or 2, and, when n is 2, L is the same or different;
   An and Ar₂ is the same or different, and each represent a substituted or unsubstituted aromatic hydrocarbon group, a substituted or unsubstituted aromatic heterocyclic group, or a substituted or unsubstituted fused polycyclic aromatic group;
   X represents an oxygen atom, a sulfur atom, or a nitrogen atom having a substituent; and
   L and Ar₁ are optionally bonded to each other to form a ring via a single bond or a linking group selected from a substituted or unsubstituted methylene group, an oxygen atom, a sulfur atom, and a nitrogen atom having a substituent; and the same holds true for Ar₁ and Ar₂.
2) The present invention is further directed to the arylamine compound having a fluorenyl skeleton-containing heterocycle structure as set forth in 1), in which R₁₀ and R₁₁ in the general formula (A) above are each a substituted or unsubstituted methyl group or a substituted or unsubstituted phenyl group.
3) The present invention is further directed to the arylamine compound having a fluorenyl skeleton-containing heterocycle structure as set forth in 1) or 2), in which X in the general formula (A) above is an oxygen atom.
4) The present invention is further directed to the arylamine compound having a fluorenyl skeleton-containing heterocycle structure as set forth in any one of 1) to 3), in which L in the general formula (A) above is a divalent group of a substituted or unsubstituted phenylene group, a substituted or unsubstituted biphenylene group, or a substituted or unsubstituted naphthylene group.
5) The present invention is further directed to the arylamine compound having a fluorenyl skeleton-containing heterocycle structure as set forth in any one of 1) to 4), in which n in the general formula (A) above is 1.
6) The present invention is further directed to an organic EL device including a pair of electrodes and one or more organic layers sandwiched therebetween, in which at least one of the organic layers contains the arylamine compound having a fluorenyl skeleton-containing heterocycle structure as set forth in any one of 1) to 5).
7) The present invention is further directed to the organic EL device as set forth in 6), in which the organic layer is an electron-blocking layer.
8) The present invention is further directed to the organic EL device as set forth in 6), in which the organic layer is a hole-transporting layer.
9) The present invention is further directed to the organic EL device as set forth in 6), in which the organic layer is a hole-injecting layer.
10) The present invention is further directed to the organic EL device as set forth in 6), in which the organic layer is a light-emitting layer.
11) The present invention is further directed to an electronic apparatus including an electronic component having a pair of electrodes and one or more organic layers sandwiched therebetween, in which at least one of the organic layers contains the arylamine compound having a fluorenyl skeleton-containing heterocycle structure as set forth in any one of 1) to 5).

Specific examples of the "linear or branched alkyl group having 1 to 6 carbon atoms", the "cycloalkyl group having 5 to 10 carbon atoms", or the "linear or branched alkenyl group having 2 to 6 carbon atoms" in the "linear or branched alkyl group having 1 to 6 carbon atoms and optionally having a substituent", the "cycloalkyl group having 5 to 10 carbon atoms and optionally having a substituent", or the "linear or branched alkenyl group having 2 to 6 carbon atoms and optionally having a substituent" represented by R₁ to R₁₁ in the general formula (A) include a methyl group, an ethyl group, an n-propyl group, an isopropyl group, an n-butyl group, an isobutyl group, a tert-butyl group, an n-pentyl group, an isopentyl group, a neopentyl group, an n-hexyl group, a cyclopentyl group, a cyclohexyl group, a 1-adamantyl group, a 2-adamantyl group, a vinyl group, an allyl group, an isopropenyl group, and a 2-butenyl group.

Specific examples of the "substituent" in the "linear or branched alkyl group having 1 to 6 carbon atoms and optionally having a substituent", the "cycloalkyl group having 5 to 10 carbon atoms and optionally having a substituent", or the "linear or branched alkenyl group having 2 to 6 carbon atoms and optionally having a substituent" represented by R₁ to R₁₁ in the general formula (A) include: a deuterium atom, a cyano group, and a nitro group; halogen atoms such as a fluorine atom, a chlorine atom, a bromine atom, and an iodine atom; silyl groups such as a trimethylsilyl group and a triphenylsilyl group; linear or branched alkyl groups having 1 to 6 carbon atoms such as a methyl group, an ethyl group, and a propyl group; linear or branched alkyloxy groups having 1 to 6 carbon atoms such as a methyloxy group, an ethyloxy group, and a propyloxy group; alkenyl groups such as a vinyl group and an allyl group; aryloxy groups such as a phenyloxy group and a tolyloxy group; arylalkyloxy groups such as a benzyloxy group and a phenethyloxy group; aromatic hydrocarbon groups or fused polycyclic aromatic groups such as a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, and a triphenylenyl group; and aromatic heterocyclic groups such as a pyridyl group, a thienyl group, a furyl group, a pyrrolyl group, a quinolyl group, an isoquinolyl group, a benzofuranyl group, a benzothienyl group, an indolyl group, a carbazolyl group, a benzooxazolyl group, a benzothiazolyl group, a quinoxalinyl group, a benzimidazolyl group, a pyrazolyl group, a dibenzofuranyl group, a dibenzothienyl group, and a carbolinyl group. These substituents may further be substituted with any of the substituents listed above. Benzene rings each substituted with the substituent, or a plurality of substituents on the same benzene ring may be bonded to each other to form a ring via a single bond or a linking group selected from a substituted or unsubstituted methylene group, an oxygen atom, a sulfur atom, and a nitrogen atom having a substituent.

Specific examples of the "linear or branched alkyloxy group having 1 to 6 carbon atoms" or the "cycloalkyloxy group having 5 to 10 carbon atoms" in the "linear or branched alkyloxy group having 1 to 6 carbon atoms and optionally having a substituent" or the "cycloalkyloxy group having 5 to 10 carbon atoms and optionally having a substituent" represented by R₁ to R₁₁ in the general formula (A) include a methyloxy group, an ethyloxy group, an n-propyloxy group, an isopropyloxy group, an n-butyloxy group, a tert-butyloxy group, an n-pentyloxy group, an n-hexyloxy group, a cyclopentyloxy group, a cyclohexyloxy group, a cycloheptyloxy group, a cyclooctyloxy group, a 1-adamantyloxy group, and a 2-adamantyloxy group.

Examples of the "substituent" in the "linear or branched alkyloxy group having 1 to 6 carbon atoms and optionally having a substituent" or the "cycloalkyloxy group having 5 to 10 carbon atoms and optionally having a substituent" represented by R₁ to R₁₁ in the general formula (A) include those listed above as examples of the "substituent" in the "linear or branched alkyl group having 1 to 6 carbon atoms and optionally having a substituent", the "cycloalkyl group having 5 to 10 carbon atoms and optionally having a substituent", or the "linear or branched alkenyl group having 2 to 6 carbon atoms and optionally having a substituent" represented by R₁ to R₁₁ in the general formula (A), and the same holds true for the forms that these substituents may be in.

Specific examples of the "aromatic hydrocarbon group", the "aromatic heterocyclic group", or the "fused polycyclic aromatic group" in the "substituted or unsubstituted aromatic hydrocarbon group", the "substituted or unsubstituted aromatic heterocyclic group", or the "substituted or unsubstituted fused polycyclic aromatic group" represented by R₁ to R₁₁ in the general formula (A) include a phenyl group, a biphenylyl group, a terphenylyl group, a naphthyl group, an anthracenyl group, a phenanthrenyl group, a fluorenyl group, a spirobifluorenyl group, an indenyl group, a pyrenyl group, a perylenyl group, a fluoranthenyl group, a triphenylenyl group, a pyridyl group, a pyrimidinyl group, a triazinyl group, a furyl group, a pyrrolyl group, a thienyl group, a quinolyl group, an isoquinolyl group, a benzofuranyl group, a benzothienyl group, an indolyl group, a carbazolyl group, a benzooxazolyl group, a benzothiazolyl group, an azafluorenyl group, a diazafluorenyl group, an azaspirobifluorenyl group, a diazaspirobifluorenyl group, a quinoxalinyl group, a benzimidazolyl group, a pyrazolyl group, a dibenzofuranyl group, a dibenzothienyl group, a naphthyridinyl group, a phenanthrolinyl group, an acridinyl group, and a carbolinyl group, and also an aryl group having 6 to 30 carbon atoms and a heteroaryl group having 2 to 20 carbon atoms.

Examples of the "substituent" in the "substituted aromatic hydrocarbon group", the "substituted aromatic heterocyclic group", or the "substituted fused polycyclic aromatic group" represented by R₁ to R₁₁ in the general formula (A) include those listed above as examples of the "substituent" in the "linear or branched alkyl group having 1 to 6 carbon atoms and optionally having a substituent", the "cycloalkyl group having 5 to 10 carbon atoms and optionally having a substituent", or the "linear or branched alkenyl group having 2 to 6 carbon atoms and optionally having a substituent" represented by R₁ to R₁₁ in the general formula (A), and the same holds true for the forms that these substituents may be in.

Specific examples of the "aryloxy group" in the "substituted or unsubstituted aryloxy group" represented by R₁ to R₁₁ in the general formula (A) include a phenyloxy group, a biphenylyloxy group, a terphenylyloxy group, a naphthyloxy group, an anthracenyloxy group, a phenanthrenyloxy group, a fluorenyloxy group, an indenyloxy group, a pyrenyloxy group, and a perylenyloxy group.

Examples of the "substituent" in the "substituted aryloxy group" represented by R₁ to R₁₁ in the general formula (A) include those listed above as examples of the "substituent" in the "linear or branched alkyl group having 1 to 6 carbon atoms and optionally having a substituent", the "cycloalkyl group having 5 to 10 carbon atoms and optionally having a substituent", or the "linear or branched alkenyl group having 2 to 6 carbon atoms and optionally having a substituent" represented by R₁ to R₁₁ in the general formula (A), and the same holds true for the forms that these substituents may be in.

In view of stability in the form of a thin film, R₁ to R₉ in the general formula (A) are each preferably a hydrogen atom, a deuterium atom, or a substituted or unsubstituted phenyl group, and more preferably a hydrogen atom, a deuterium atom, or an unsubstituted phenyl group.

In view of heat resistance of the compound, R₁₀ and R₁₁ in the general formula (A) are each preferably a substituted or an unsubstituted methyl group or a substituted or unsubstituted phenyl group, and more preferably an unsubstituted methyl group or an unsubstituted phenyl group. R₁₀ and R₁₁ are preferably the same.

Examples of the "aromatic hydrocarbon group", the "aromatic heterocyclic group", or the "fused polycyclic aromatic group" in the "substituted or unsubstituted aromatic hydrocarbon group", the "substituted or unsubstituted aromatic heterocyclic group", or the "substituted or unsubstituted fused polycyclic aromatic group" represented by L in the general formula (A) include groups obtained by removing one hydrogen atom from those listed above as examples of the "aromatic hydrocarbon group", the "aromatic heterocyclic group", or the "fused polycyclic aromatic group" in the "substituted or unsubstituted aromatic hydrocarbon group", the "substituted or unsubstituted aromatic heterocyclic group", or the "substituted or unsubstituted fused polycyclic aromatic group" represented by R₁ to R₁₁ in the general formula (A).

Examples of the "substituent" in the "substituted aromatic hydrocarbon group", the "substituted aromatic heterocyclic group", or the "substituted fused polycyclic aromatic group" represented by L in the general formula (A) include those listed above as examples of the "substituent" in the "linear or branched alkyl group having 1 to 6 carbon atoms and optionally having a substituent", the "cycloalkyl group having 5 to 10 carbon atoms and optionally having a substituent", or the "linear or branched alkenyl group having 2 to 6 carbon atoms and optionally having a substituent" represented by R₁ to R₁₁ in the general formula (A), and the same holds true for the forms that these substituents may be in.

In view of hole-transporting capability and electron-blocking capability, L in the general formula (A) is preferably a substituted or unsubstituted phenylene group, a substituted or unsubstituted biphenylene group, or a substituted or unsubstituted naphthylene group, more preferably an unsubstituted phenylene group, an unsubstituted biphenylene group, or an unsubstituted naphthylene group, and even more preferably an unsubstituted phenylene group or an unsubstituted naphthylene group.
n in the general formula (A) is preferably 1.

Examples of the "aromatic hydrocarbon group", the "aromatic heterocyclic group", or the "fused polycyclic aromatic group" in the "substituted or unsubstituted aromatic hydrocarbon group", the "substituted or unsubstituted aromatic heterocyclic group", or the "substituted or unsubstituted fused polycyclic aromatic group" represented by Ar₁ and Ar₂ in the general formula (A) include those listed above as examples of the "aromatic hydrocarbon group", the "aromatic heterocyclic group", or the "fused polycyclic aromatic group" in the "substituted or unsubstituted aromatic hydrocarbon group", the "substituted or unsubstituted aromatic heterocyclic group", or the "substituted or unsubstituted fused polycyclic aromatic group" represented by R₁ to R₁₁ in the general formula (A).

Examples of the "substituent" in the "substituted aromatic hydrocarbon group", the "substituted aromatic heterocyclic group", or the "substituted fused polycyclic aromatic group" represented by Ar₁ and Ar₂ in the general formula (A) include those listed above as examples of the "substituent" in the "linear or branched alkyl group having 1 to 6 carbon atoms and optionally having a substituent", the "cycloalkyl group having 5 to 10 carbon atoms and optionally having a substituent", or the "linear or branched alkenyl group having 2 to 6 carbon atoms and optionally having a substituent" represented by R₁ to R₁₁ in the general formula (A), and the same holds true for the forms that these substituents may be in.

In view of hole-transporting capability and electron-blocking capability, Ar₁ and Ar₂ in the general formula (A) are each preferably an unsubstituted phenyl group or an unsubstituted biphenylyl group.

In view of hole-transporting capability and electron-blocking capability, X in the general formula (A) is preferably an oxygen atom.

The arylamine compound having a fluorenyl skeleton-containing heterocycle structure represented by the general formula (A) above, which is suitable for use in an organic EL device of the present invention, is preferably used as a material for forming a hole-injecting layer, a hole-transporting layer, an electron-blocking layer, or a light-emitting layer of the organic EL device, and more preferably used as a material for forming the hole-transporting layer or the electron-blocking layer.

The arylamine compound having a fluorenyl skeleton-containing heterocycle structure of the present invention has the following properties: (1) better hole-injecting performance, (2) higher hole mobility, (3) excellent electron-blocking capability, (4) higher electron resistance, (5) higher stability in the form of a thin film, and (6) excellent heat resistance, compared with those of conventional hole-transporting materials. When the arylamine compound having a fluorenyl skeleton-containing heterocycle structure of the present invention is used for an organic EL device, the resulting organic EL device has the following properties: (7) high luminous efficacy, (8) a low voltage for the start of light emission, (9) a low actual driving voltage, and (10) a long lifespan.

The arylamine compound having a fluorenyl skeleton-containing heterocycle structure of the present invention is excellent in terms of hole-injecting/transporting performance, stability in the form of a thin film, and durability. Accordingly, the organic EL device having a hole-injecting layer and/or a hole-transporting layer formed by using this compound as a hole injection material and/or a hole-transporting material has higher efficiency in transporting holes to the light-emitting layer, and therefore higher luminous efficacy, and further has a lower driving voltage, and therefore improved device durability. Thus, the device has the improved properties including high efficiency, a low driving voltage, and a long lifespan.

The arylamine compound having a fluorenyl skeleton-containing heterocycle structure of the present invention has excellent electron-blocking capability, high electron resistance, and high stability in the form of a thin film, and can confine excitons generated in the light-emitting layer. Thus, the organic EL device having an electron-blocking layer formed by using this compound as an electron-blocking material has an increased probability of recombinations of holes and electrons to prevent thermal deactivation, and therefore has a higher luminous efficacy, and the organic EL device also has a lower driving voltage and therefore higher current resistance, and accordingly has an increased maximum luminance.

The arylamine compound having a fluorenyl skeleton-containing heterocycle structure of the present invention has excellent hole-transporting capability and a wide bandgap. Accordingly, the organic EL device having a light-emitting layer formed by using this compound as a host material and doping thereinto a so-called dopant, such as a fluorescent emitter, a phosphorescent emitter, or a delayed fluorescent emitter, has a lower driving voltage and a higher luminous efficacy.

Accordingly, the arylamine compound having a fluorenyl skeleton-containing heterocycle structure of the present invention is useful as materials of a hole-injecting layer, a hole-transporting layer, an electron-blocking layer, or a light-emitting layer of an organic EL device, and can improve the luminous efficacy, the driving voltage, and the durability, compared with those of conventional organic EL devices.

### Brief Description of Drawings

[Fig. 1] Compounds of (1) to (15) as preferred specific examples of the arylamine compound represented by the general formula (A).
[Fig. 2] Compounds of (16) to (30) as preferred specific examples of the arylamine compound represented by the general formula (A).
[Fig. 3] Compounds of (31) to (45) as preferred specific examples of the arylamine compound represented by the general formula (A).
[Fig. 4] Compounds of (46) to (60) as preferred specific examples of the arylamine compound represented by the general formula (A).
[Fig. 5] Compounds of (61) to (75) as preferred specific examples of the arylamine compound represented by the general formula (A).
[Fig. 6] Compounds of (76) to (90) as preferred specific examples of the arylamine compound represented by the general formula (A).
[Fig. 7] Compounds of (91) to (105) as preferred specific examples of the arylamine compound represented by the general formula (A).
[Fig. 8] Compounds of (106) to (114) as preferred specific examples of the arylamine compound represented by the general formula (A).
[Fig. 9] A diagram showing an example of the configuration of the organic EL device of the present invention.

### Description of Embodiments

The arylamine compounds having a fluorenyl skeleton-containing heterocycle structure of the present invention are novel compounds. These compounds can be synthesized according to methods known per se (see Patent Literature 5, for example).

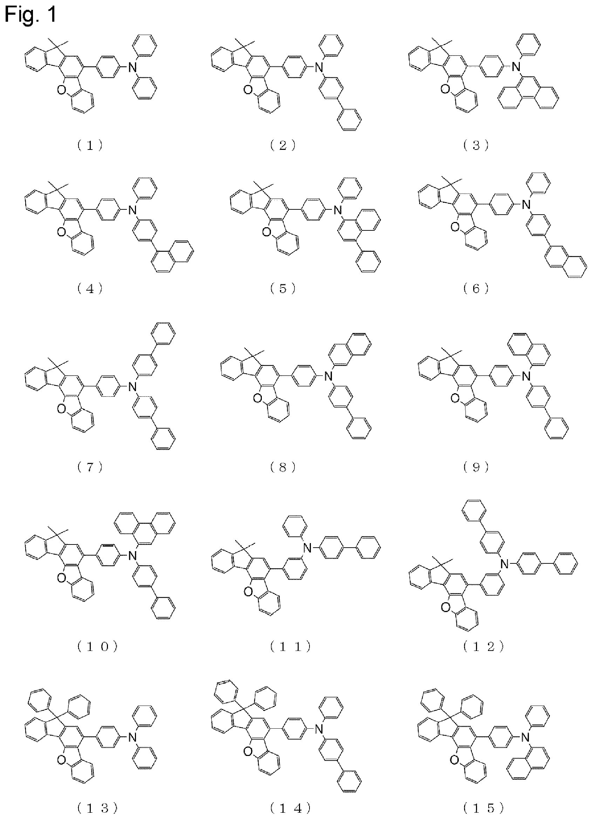

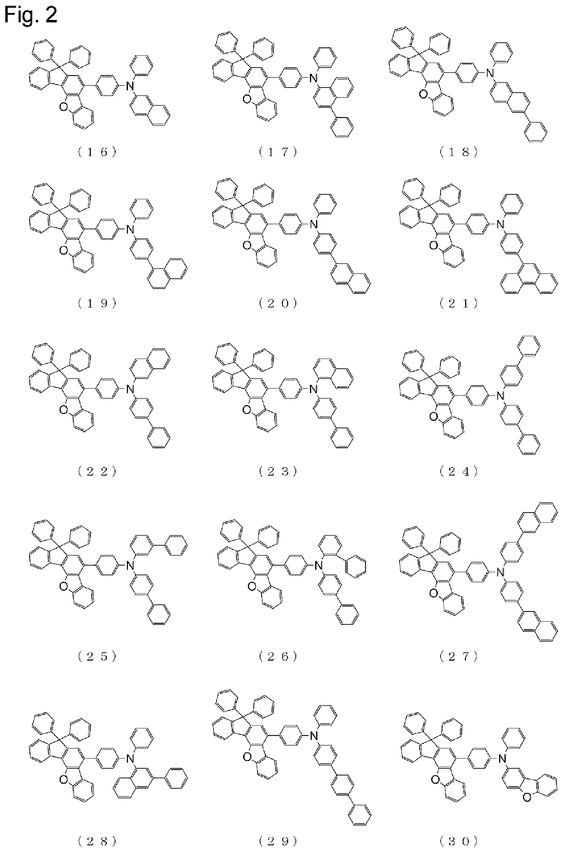

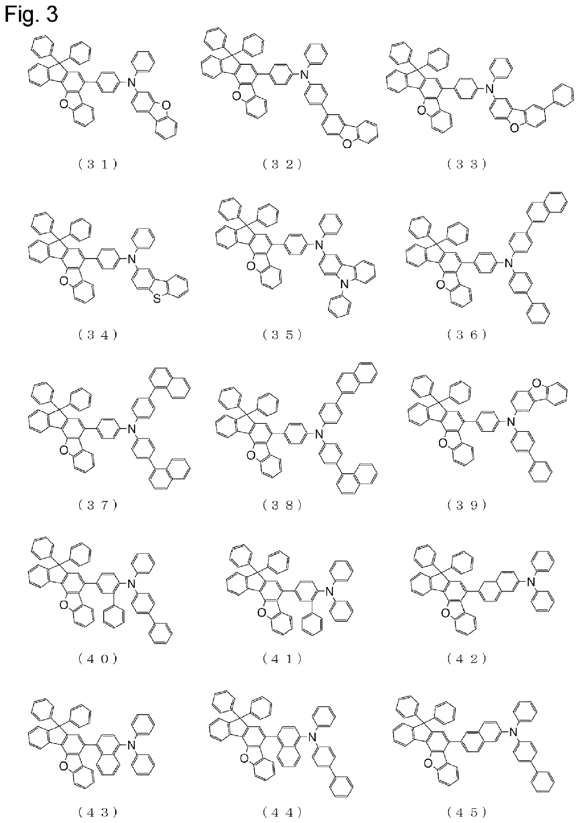

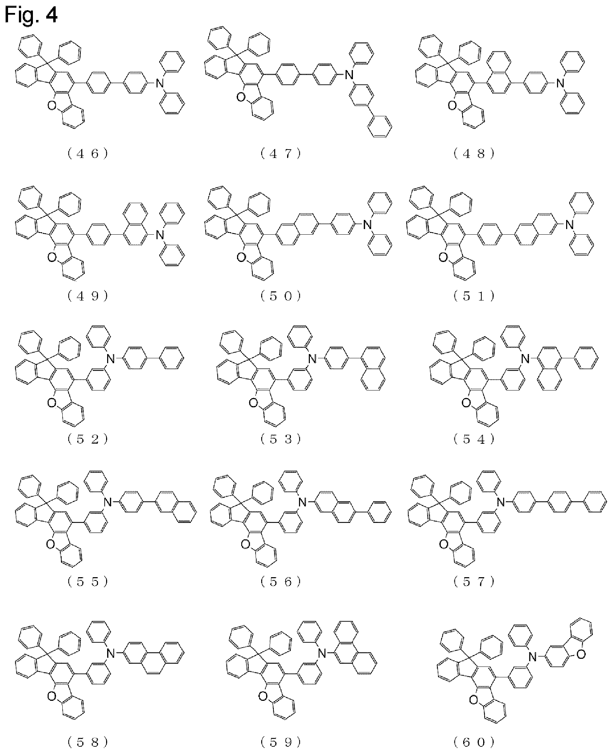

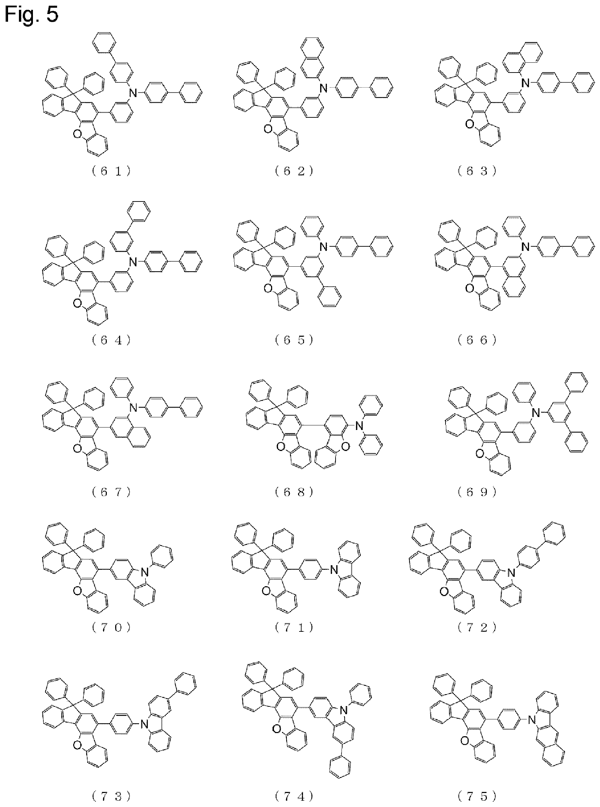

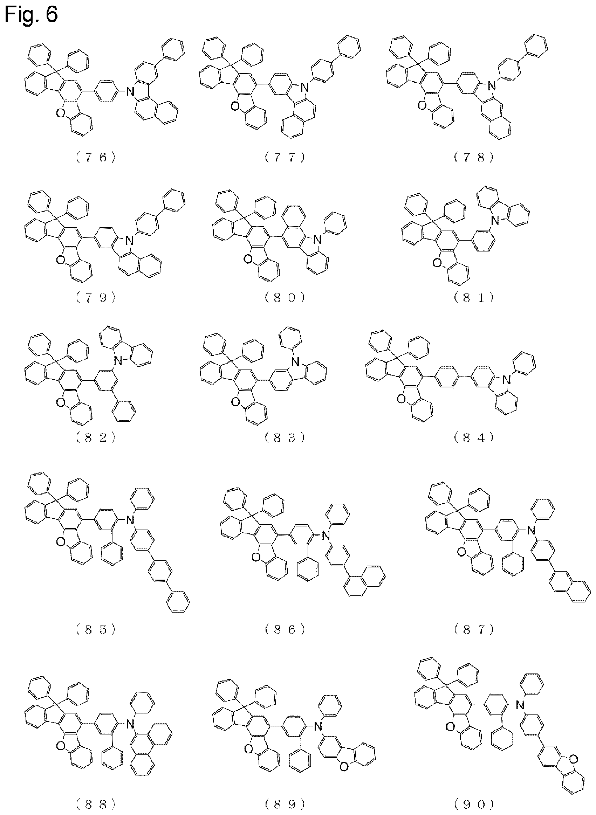

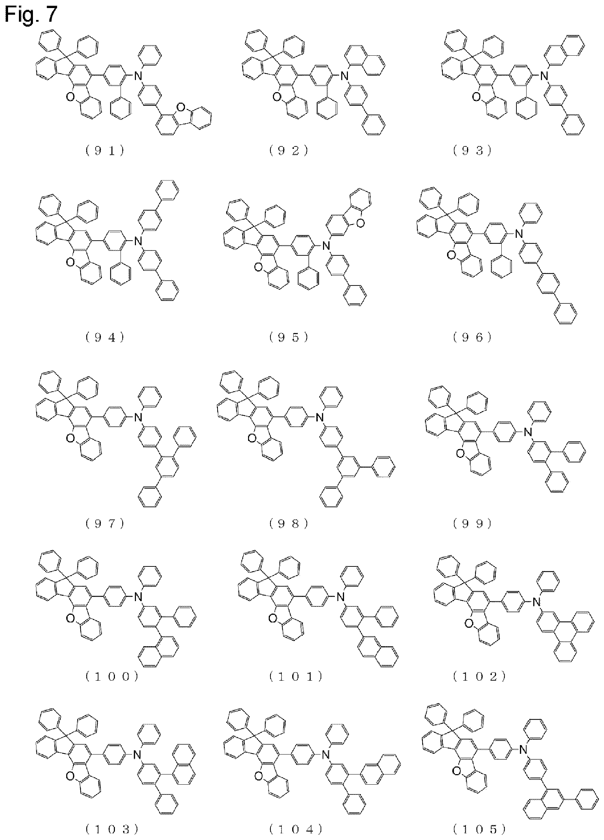

Specific preferred examples of the arylamine compound having a fluorenyl skeleton-containing heterocycle structure represented by the general formula (A) above and suitably used in an organic EL device of the present invention are shown in Figs. 1 to 8. However, the arylamine compound of the present invention is not limited thereto.

The arylamine compound represented by the general formula (A) can be purified by any known purification method, such as column chromatography, adsorption with silica gel, activated carbon, activated clay, or the like, recrystallization or crystallization from a solvent, or sublimation. The compound can be identified by NMR analysis. The physical properties can be determined in terms of the melting point, the glass transition point (Tg), the work function, and others. The melting point is a measure of the vapor deposition properties. The glass transition point (Tg) is a measure of the stability in the form of a thin film. The work function is a measure of the hole injectability, the hole-transporting capability, and the electron block ability.

The melting point and the glass transition point (Tg) can be measured, for example, on the compound in the form of a powder using a high-sensitivity differential scanning calorimeter (DSC3100SA manufactured by Bruker AXS K.K.).

The work function can be determined, for example, on the compound in the form of a thin film with a thickness of 100 nm on an ITO substrate using an ionization potential measurement system (PYS-202 manufactured by Sumitomo Heavy Industries, Ltd.).

The organic EL device of the present invention may have: a structure in which an anode, a hole-injecting layer, a hole-transporting layer, a light-emitting layer, an electron-transporting layer, an electron-injecting layer, and a cathode are sequentially formed on a substrate; and the structure may further include an electron-blocking layer between the hole-transporting layer and the light-emitting layer and/or a hole-blocking layer between the light-emitting layer and the electron-transporting layer. In these multilayer structures, a single organic layer can perform the functions of several layers. For example, a single organic layer may serve as both the hole-injecting layer and the hole-transporting layer, and a single organic layer may serve as both the electron-injecting layer and the electron-transporting layer. It is also possible to stack two or more organic layers having the same function. Specifically, two hole-transporting layers may be stacked; two light-emitting layers may be stacked; and two electron-transporting layers may be stacked.

An electrode material having a high work function, such as ITO or gold, is used for the anode of the organic EL device of the present invention. Examples of the materials used for the hole-injecting layer of the organic EL device of the present invention include porphyrin compounds typified by copper phthalocyanine; starburst triphenylamine derivatives; arylamine compounds having a structure containing two or more triphenylamine structures or carbazolyl structures in the molecule, the triphenylamine or carbazolyl structures being linked via a single bond or a divalent group having no heteroatom; acceptor type heterocyclic compounds such as hexacyanoazatriphenylene; and coating type polymer materials.

Examples of the materials used for the hole-injecting layer and the hole-transporting layer of the organic EL device of the present invention include: the arylamine compound having a fluorenyl skeleton-containing heterocycle structure of the present invention; benzidine derivatives such as N,N'-diphenyl-N,N'-di(m-tolyl)-benzidine (hereinafter abbreviated as TPD), N,N'-diphenyl-N,N'-di(α-naphthyl)-benzidine (hereinafter abbreviated as NPD), and N,N,N',N'-tetrabiphenylyl benzidine; 1,1-bis[(di-4-tolylamino)phenyl]cyclohexane (hereinafter abbreviated as TAPC); and arylamine compounds having a structure containing two or more triphenylamine structures or carbazolyl structures in the molecule, the triphenylamine or carbazolyl structures being linked via a single bond or a divalent group having no heteroatom. These materials may be used singly for film formation, or two or more of these materials may be mixed and used for film formation. In each case, a single layer may be formed. The hole-injecting layer and the hole-transporting layer may have a layered structure composed of different layers each formed of a single kind of the materials described above, a layered structure composed of different layers each formed of a mixture of the materials described above, or a layered structure composed of a layer formed of a single kind of the materials described above and a layer formed of a mixture of two or more of the materials described above. Other examples of the materials used for the hole-injecting/transporting layers include coating type polymer materials such as poly(3,4-ethylenedioxythiophene) (hereinafter abbreviated as PEDOT)/poly (styrenesulfonate) (hereinafter abbreviated as PSS).

Other examples of the materials used for the hole-injecting layer or the hole-transporting layer include a material obtained by p-doping a material normally used for these layers with trisbromophenylamine hexachloroantimony or a radialene derivative (see Patent Literature 6, for example); and a polymer compound having the structure of a benzidine derivative, such as TPD, as a partial structure thereof.

Examples of the materials used for the electron-blocking layer of the organic EL device of the present invention include: the arylamine compound having a fluorenyl skeleton-containing heterocycle structure of the present invention; and also compounds having an electron-blocking effect, such as carbazole derivatives such as 4,4',4"-tri(N-carbazolyl)triphenylamine (hereinafter abbreviated as TCTA), 9,9-bis[4-(carbazole-9-yl)phenyl]fluorene, 1,3-bis(carbazole-9-yl)benzene (hereinafter abbreviated as mCP), and 2,2-bis(4-carbazole-9-ylphenyl)adamantane (hereinafter abbreviated as Ad-Cz); and compounds having a triphenylsilyl group and a triarylamine structure such as 9-[4-(carbazole-9-yl)phenyl]-9-[4-(triphenylsilyl)phenyl]-9H-fluorene. These materials may also serve as the material used for the hole-transporting layer.

Examples of the materials of the light-emitting layer of the organic EL device of the present invention include: the arylamine compound having a fluorenyl skeleton-containing heterocycle structure of the present invention; metal complexes of quinolinol derivatives such as Alq₃; various types of metal complexes; an anthracene derivative; a bisstyrylbenzene derivative; a pyrene derivative; an oxazole derivative; and a polyphenylene vinylene derivative. The light-emitting layer may also include a host material and a dopant material. As the host material, an anthracene derivative is preferably used. Other examples of the host material include the light emitting materials including the arylamine compound of the present invention, and also a heterocyclic compound having an indole ring as a partial structure of a fused ring, a heterocyclic compound having a carbazole ring as a partial structure of a fused ring, a carbazole derivative, a thiazole derivative, a benzimidazole derivative, and a polydialkylfluorene derivative. Examples of the dopant material include quinacridone, coumalin, rubrene, perylene, and derivatives thereof; a benzopyran derivative; a rhodamine derivative; and an aminostyryl derivative.

A phosphorescent emitter can also be used as a light emitting material. The phosphorescent emitter may be a metal complex of iridium, platinum, or the like, and examples thereof include a green phosphorescent emitter such as Ir(ppy)₃, a blue phosphorescent emitter such as FIrpic or FIr6, and a red phosphorescent emitter such as Btp₂Ir (acac). As a host material for this case, a host material having hole injecting/transporting capability may be used, including carbazole derivatives such as 4,4'-di(N-carbazolyl)biphenyl (hereinafter abbreviated as CBP), TCTA, and mCP, and also the arylamine compound of the present invention. A host material having electron-transporting capability may also be used, including p-bis(triphenylsilyl)benzene (hereinafter abbreviated as UGH2) and 2,2',2"-(1,3,5-phenylene)-tris(1-phenyl-1H-benzimidazole) (hereinafter abbreviated as TPBI). Use of these materials enables production of a high-performance organic EL element.

In order to avoid concentration quenching, doping of a host material with a phosphorescent emitter is preferably performed by co-deposition in an amount within a range of 1 to 30 wt% based on the entire light-emitting layer.

As the light emitting material, a material that emits delayed fluorescence can also be used, including a CDCB derivative such as PIC-TRZ, CC2TA, PXZ-TRZ, and 4CzIPN (see Non-Patent Literature 3, for example).

Examples of the materials used for the hole blocking layer of the organic EL device of the present invention include compounds exhibiting a hole blocking effect, including a phenanthroline derivative, such as bathocuproine (hereinafter abbreviated as BCP); a metal complex of a quinolinol derivative, such as BAlq; various types of rare-earth complexes; an oxazole derivative; a triazole derivative; and a triazine derivative. These materials may also serve as the material of the electron-transporting layer.

Examples of the material used for the electron-transporting layer of the organic EL device of the present invention include metal complexes of quinolinol derivatives, such as Alq₃ and BAlq; various types of metal complexes; a triazole derivative; a triazine derivative; an oxadiazole derivative; a pyridine derivative; a pyrimidine derivative; a benzimidazole derivative; a thiadiazole derivative; an anthracene derivative; a carbodiimide derivative; a quinoxaline derivative; a pyridoindole derivative; a phenanthroline derivative; and a silole derivative.

Examples of the materials used for the electron-injecting layer of the organic EL device of the present invention include alkali metal salts such as lithium fluoride and cesium fluoride; an alkaline earth metal salt such as magnesium fluoride; a metal complex of a quinolinol derivative such as lithium quinolinol; a metal oxide such as aluminum oxide; and a metal such as ytterbium (Yb), samarium (Sm), calcium (Ca), strontium (Sr), and cesium (Cs). The electron-injecting layer can however be omitted when an electron-transporting layer and a cathode are suitably selected.

Furthermore, a material obtained by n-doping a material normally used for an electron-injecting layer or an electron-transporting layer with a metal such as cesium can be used for the electron-injecting layer or the electron-transporting layer.

For the cathode of the organic EL device of the present invention, a metal having a low work function, such as aluminum; or an alloy having an even lower work function, such as a magnesium-silver alloy, a magnesium-indium alloy, or an aluminum-magnesium alloy is used as the electrode material.

These materials for the layers in the organic EL device may be used in a known method, such as vapor deposition, spin coating, or inkjet printing, to form a thin film. These materials may be used singly for film formation, or two or more of these materials may be mixed and used for film formation. In each case, a single layer film may be formed. The film may have a layered structure composed of different layers each formed of a single kind of the materials described above, a layered structure composed of different layers each formed of a mixture of the materials described above, or a layered structure composed of a layer formed of a single kind of the materials described above and a layer formed of a mixture of two or more of the materials described above.

### Examples

Hereinafter, embodiments of the present invention will be described in greater detail by way of examples. However, the present invention is not limited thereto as long as it does not depart from the gist thereof.

### Example 1

### <Synthesis of bis(biphenyl-4-yl)-{3-(7,7-dimethyl-7H-12-oxa-indeno[1,2-a]fluorene-5-yl)-phenyl}-amine (Compound (12))>

First, 7.0 g of 5-bromo-7,7-dimethyl-7H-12-oxa-indeno[1,2-a]fluorene, 12.1 g of bis(biphenyl-4-yl)-{3-(4,4,5,5-tetramethyl-[1,3,2]dioxaborolane-2-yl)-phenyl}-amine, 0.3 g of [1,1'-bis(diphenylphosphino)ferrocene]dichloropalladium(II), and 2.4 g of sodium hydrogencarbonate were placed in a reaction vessel and stirred in a THF/H₂O mixed solvent overnight under reflux. After allowing to cool, ethyl acetate/H₂O was added to the system, and the organic layer was obtained by extraction and partition and concentrated to obtain a crude product. The obtained crude product was purified through crystallization from a dichloromethane/acetone mixed solvent to obtain 12.2 g of a white powder of bis(biphenyl-4-yl)-{3-(7,7-dimethyl-7H-12-oxa-indeno[1,2-a]fluorene-5-yl)-phenyl}-amine (Compound (12)) (yield: 92.9%).

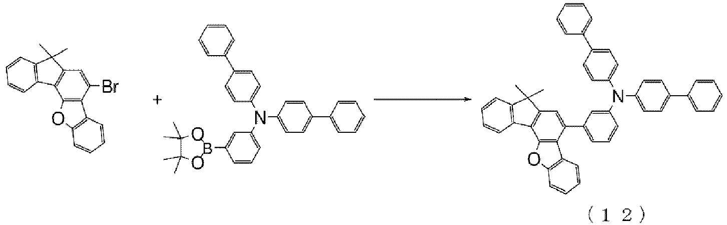

The obtained white powder was analyzed by ¹H-NMR (CDCl₃), and as a result, the following 37 hydrogen signals were detected to identify the structure thereof.
δ (ppm) = 8.30 (1H), 7.68 (1H), 7.62 (1H), 7.61-7.50 (11H), 7.49 (1H), 7.44 (6H), 7.39 (2H), 7.33 (7H), 7.18 (1H), 1.61 (6H).

### Example 2

### <Synthesis of biphenyl-4-yl-{4-(7,7-diphenyl-7H-12-oxa-indeno[1,2-a]fluorene-5-yl)-phenyl}-phenyl-amine (Compound (14))>

First, 10.0 g of 5-bromo-7,7-diphenyl-7H-12-oxa-indeno[1,2-a]fluorene, 9.0 g of 4-(biphenyl-4-yl-phenyl-amino)-phenylboronic acid, 0.3 g of [1,1'-bis(diphenylphosphino)ferrocene]dichloropalladium(II), and 3.5 g of sodium hydrogencarbonate were placed in a reaction vessel and stirred in a THF/H₂O mixed solvent overnight under reflux. After allowing to cool, ethyl acetate/H₂O was added to the system, and the organic layer was obtained by extraction and partition and concentrated to obtain a crude product. The obtained crude product was purified through crystallization from a dichloromethane/acetone mixed solvent to obtain 8.5 g of a pale yellow powder of biphenyl-4-yl-{4-(7,7-diphenyl-7H-12-oxa-indeno[1,2-a]fluorene-5-yl)-phenyl}-phenyl-amine (Compound (14)) (yield: 56.9%).

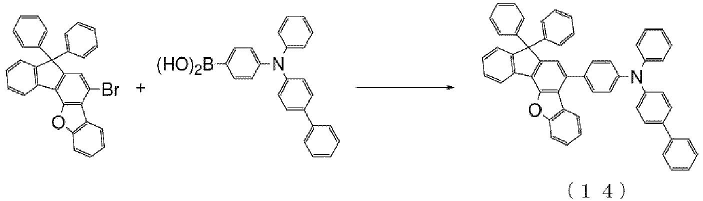

The obtained pale yellow powder was analyzed by ¹H-NMR (CDCl₃), and as a result, the following 37 hydrogen signals were detected to identify the structure thereof.
δ (ppm) = 8.38 (1H), 7.78 (1H), 7.73 (1H), 7.63 (2H), 7.56 (2H), 7.49 (7H), 7.39-7.20 (22H), 7.11 (1H).

### Example 3

### <Synthesis of biphenyl-4-yl-{3-(7,7-diphenyl-7H-12-oxa-indeno[1,2-a]fluorene-5-yl)-phenyl}-phenyl-amine (Compound (52))>

First, 7.0 g of 5-bromo-7,7-diphenyl-7H-12-oxa-indeno[1,2-a]fluorene, 7.4 g of biphenyl-4-yl-phenyl-{3-(4,4,5,5-tetramethyl-[1,3,2]dioxaborolane-2-yl)-phenyl}-amine, 0.2 g of [1,1'-bis(diphenylphosphino)ferrocene]dichloropalladium(II), and 2.4 g of sodium hydrogencarbonate were placed in a reaction vessel and stirred in a THF/H₂O mixed solvent overnight under reflux. After allowing to cool, ethyl acetate/H₂O was added to the system, and the organic layer was obtained by extraction and partition and concentrated to obtain a crude product. The obtained crude product was purified through recrystallization from an acetone solvent to obtain 8.5 g of a white powder of biphenyl-4-yl-{3-(7,7-diphenyl-7H-12-oxa-indeno[1,2-a]fluorene-5-yl)-phenyl}-phenyl-amine (Compound (52)) (yield: 81.3%).

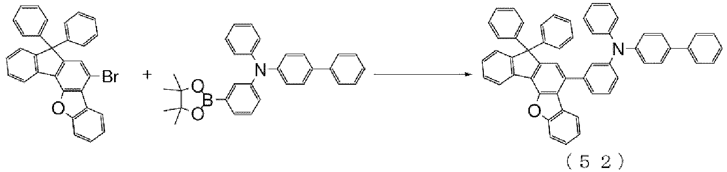

The obtained white powder was analyzed by ¹H-NMR (CDCl₃), and as a result, the following 37 hydrogen signals were detected to identify the structure thereof.
δ (ppm) = 8.35 (1H), 7.69 (1H), 7.62 (1H), 7.57 (2H), 7.54-7.19 (30H), 7.17 (1H), 7.06 (1H).

### Example 4

### <Synthesis of bis(biphenyl-4-yl)-{4-(7,7-dimethyl-7H-12-oxa-indeno[1,2-a]fluorene-5-yl)-phenyl}-amine (Compound (7))>

First, 4.0 g of 5-(4-chlorophenyl)-7,7-dimethyl-7H-12-oxa-indeno[1,2-a]fluorene, 4.2 g of bis(biphenyl-4-yl)-amine, 0.1 g of bis(tri-t-butylphosphine)palladium(0), and 3.0 g of sodium t-butoxide were placed in a reaction vessel and stirred in a toluene solvent overnight under reflux. After allowing to cool, the resulting system was filtered, and the filtrate was concentrated to obtain a crude product. The obtained crude product was purified through crystallization from a dichloromethane/acetone mixed solvent to obtain 5.2 g of a yellow powder of bis(biphenyl-4-yl)-{4-(7,7-dimethyl-7H-12-oxa-indeno[1,2-a]fluorene-5-yl)-phenyl}-amine (Compound (7)) (yield: 75.4%).

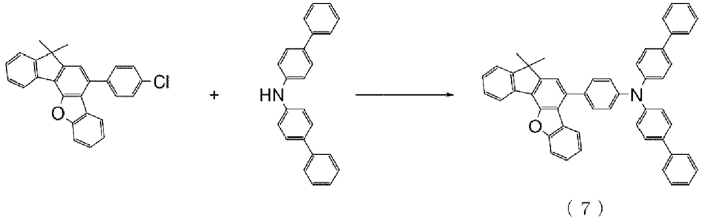

The obtained yellow powder was analyzed by ¹H-NMR (CDCl₃), and as a result, the following 37 hydrogen signals were detected to identify the structure thereof.
δ (ppm) = 8.29 (1H), 7.74 (1H), 7.69 (1H), 7.62 (6H), 7.58 (4H), 7.51-7.41 (7H), 7.39-7.30 (10H), 7.22 (1H), 1.60 (6H).

### Example 5

### <Synthesis of bis(biphenyl-4-yl)-{4-(7,7-diphenyl-7H-12-oxa-indeno[1,2-a]fluorene-5-yl)-phenyl}-amine (Compound (24))>

First, 8.1 g of 5-(4-chlorophenyl)-7,7-diphenyl-7H-12-oxa-indeno[1,2-a]fluorene, 5.5 g of bis(biphenyl-4-yl)-amine, 0.2 g of bis(tri-t-butylphosphine)palladium(0), and 3.0 g sodium t-butoxide were placed in a reaction vessel and stirred in a toluene solvent overnight under reflux. After allowing to cool, the resulting system was filtered, and the filtrate was concentrated to obtain a crude product. The obtained crude product was purified through crystallization from a monochlorobenzene/acetone mixed solvent to obtain 9.8 g of a white powder of bis(biphenyl-4-yl)-{4-(7,7-diphenyl-7H-12-oxa-indeno[1,2-a]fluorene-5-yl)-phenyl}-amine (Compound (24)) (yield: 78.1%).

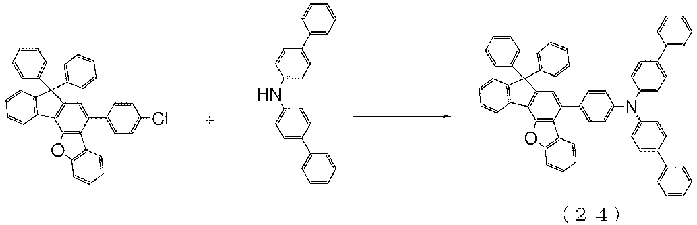

The obtained white powder was analyzed by ¹H-NMR (CDCl₃), and as a result, the following 41 hydrogen signals were detected to identify the structure thereof.
δ (ppm) = 8.35 (1H), 7.75 (1H), 7.71 (1H), 7.60 (4H), 7.55 (4H), 7.51 (2H), 7.49-7.40 (7H), 7.34 (2H), 7.33-7.26 (12H), 7.25-7.18 (7H).

### Example 6

### <Synthesis of (biphenyl-2-yl)-(biphenyl-4-yl)-{4-(7,7-dimethyl-7H-12-oxa-indeno[1,2-a]fluorene-5-yl)-phenyl}-amine (Compound (26))>

First, 9.0 g of (biphenyl-4-yl)-(biphenyl-4-yl)-{4-(7,7-dimethyl-7H-12-oxa-indeno[1,2-a]fluorene-5-yl)-phenyl}-amine, 3.9 g of 2-bromobiphenyl, 0.1 g of palladium(II) acetate, 0.2 g of tri-t-butylphosphine, and 2.0 g sodium t-butoxide were placed in a reaction vessel and stirred in a toluene solvent for 3 hours under reflux. After allowing to cool, the resulting system was filtered, and the filtrate was concentrated to obtain a crude product. The obtained crude product was purified by column chromatography (column: silica gel, eluent: dichloromethane/n-heptane) to obtain 7.6 g of a white powder of (biphenyl-2-yl)-(biphenyl-4-yl)-{4-(7,7-dimethyl-7H-12-oxa-indeno[1,2-a]fluorene-5-yl)-phenyl}-amine (Compound (26)) (yield: 68.4%).

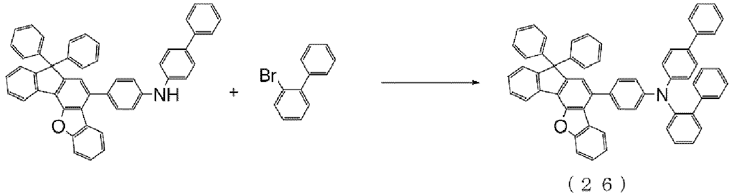

The obtained white powder was analyzed by ¹H-NMR (CDCl₃), and as a result, the following 41 hydrogen signals were detected to identify the structure thereof.
δ (ppm) = 8.33 (1H), 7.68 (1H), 7.55 (2H), 7.51 (1H), 7.50-7.38 (10H), 7.37-7.08 (24H), 6.91 (2H).

### Example 7

### <Synthesis of diphenyl-{1-(7,7-diphenyl-7H-12-oxa-indeno[1,2-a]fluorene-5-yl)-naphthalene-4-yl}-amine (Compound (43))>

First, 8.7 g of 5-(4,4,5,5-tetramethyl-[1,3,2]dioxaborolane-2-yl)-7,7-diphenyl-7H-12-oxa-indeno[1,2-a]fluorene, 6.0 g of diphenylamino-naphthalene-4-yl-trifluoromethanesulfonate, 0.2 g of [1,1'-bis(diphenylphosphino)ferrocene]dichloropalladium(II), and 1.7 g sodium hydrogencarbonate were placed in a reaction vessel and stirred in a THF/H₂O mixed solvent overnight under reflux. After allowing to cool, methanol was added to the system to form a solid precipitate, and the precipitate was collected as a crude product by filtration. The obtained crude product was purified through crystallization from a toluene/acetone mixed solvent to obtain 4.4 g of a pale yellow powder of diphenyl-{1-(7,7-diphenyl-7H-12-oxa-indeno[1,2-a]fluorene-5-yl)-naphthalene-4-yl}-amine (Compound (43)) (yield: 45.9%).

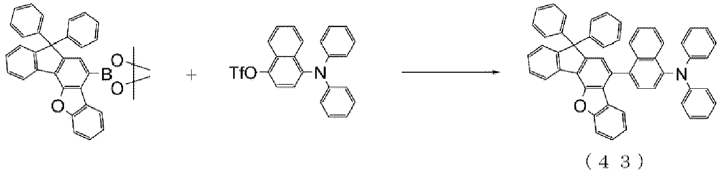

The obtained pale yellow powder was analyzed by ¹H-NMR (CDCl₃), and as a result, the following 35 hydrogen signals were detected to identify the structure thereof.
δ (ppm) = 8.40 (1H), 8.07 (1H), 7.68 (1H), 7.61 (1H), 7.56 (1H), 7.52 (1H), 7.50 (1H), 7.46 (1H), 7.42 (1H), 7.40-7.17 (18H), 7.13 (4H), 6.97 (3H), 6.71 (1H).

### Example 8

### <Synthesis of bis(biphenyl-4-yl)-{3-(7,7-diphenyl-7H-12-oxa-indeno[1,2-a]fluorene-5-yl)-phenyl}-amine (Compound (61))>

First, 10.0 g of 5-bromo-7,7-diphenyl-7H-12-oxa-indeno[1,2-a]fluorene, 12.9 g of bis(biphenyl-4-yl)-{3-(4,4,5,5-tetramethyl-[1,3,2]dioxaborolane-2-yl)-phenyl}-amine, 0.3 g of [1,1'-bis(diphenylphosphino)ferrocene]dichloropalladium(II), and 2.6 g sodium hydrogencarbonate were placed in a reaction vessel and stirred in a THF/H₂O mixed solvent for 2 hours under reflux. After allowing to cool, ethyl acetate/H₂O was added to the system, and the organic layer was obtained by extraction and partition and concentrated to obtain a crude product. The obtained crude product was purified through crystallization from a dichloromethane/acetone mixed solvent to obtain 14.5 g of a white powder of bis(biphenyl-4-yl)-{3-(7,7-diphenyl-7H-12-oxa-indeno[1,2-a]fluorene-5-yl)-phenyl}-amine (Compound (61)) (with a yield of 87.9%).

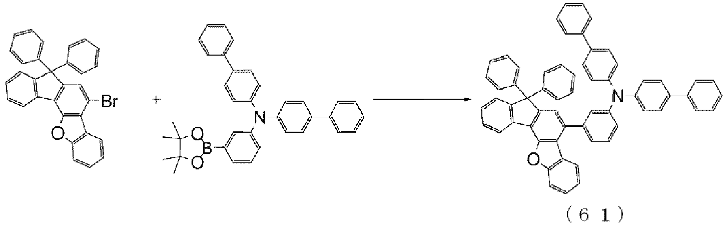

The obtained white powder was analyzed by ¹H-NMR (CDCl₃), and as a result, the following 41 hydrogen signals were detected to identify the structure thereof.
δ (ppm) = 8.32 (1H), 7.66 (1H), 7.60 (1H), 7.55 (4H), 7.49 (4H), 7.48-7.38 (8H), 7.35 (2H), 7.32-7.26 (5H), 7.24 (8H), 7.21-7.12 (7H).

### Example 9

### <Synthesis of bis(biphenyl-4-yl)-{5-(7,7-diphenyl-7H-12-oxa-indeno[1,2-a]fluorene-5-yl)-biphenyl-2-yl}-amine (Compound (94))>

First, 4.3 g of 5-bromo-7,7-diphenyl-7H-12-oxa-indeno[1,2-a]fluorene, 6.3 g of bis(biphenyl-4-yl)-{5-(4,4,5,5-tetramethyl-[1,3,2]dioxaborolane-2-yl)-biphenyl-2-yl}-amine, 0.1 g of [1,1'-bis(diphenylphosphino)ferrocene]dichloropalladium(II), and 1.1 g sodium hydrogencarbonate were placed in a reaction vessel and stirred in a THF/H₂O mixed solvent for 5 hours under reflux. After allowing to cool, methanol was added to the system to form a solid precipitate, and the precipitate was collected as a crude product by filtration. The obtained crude product was purified through crystallization from a dichloromethane/acetone mixed solvent to obtain 7.1 g of a pale yellow powder of bis(biphenyl-4-yl)-{5-(7,7-diphenyl-7H-12-oxa-indeno[1,2-a]fluorene-5-yl)-biphenyl-2-yl}-amine (Compound (94)) (yield: 91.6%).

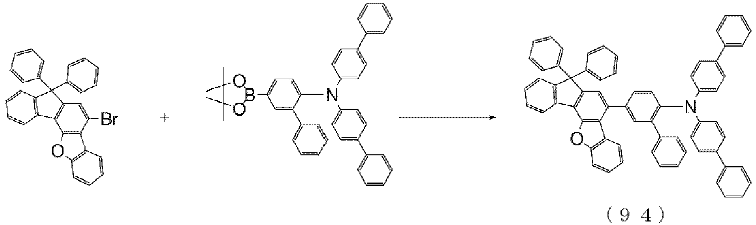

The obtained pale yellow powder was analyzed by ¹H-NMR (CDCl₃), and as a result, the following 45 hydrogen signals were detected to identify the structure thereof.
δ (ppm) = 8.37 (1H), 7.79 (1H), 7.72 (1H), 7.62 (1H), 7.60 (1H), 7.53 (5H), 7.48 (3H), 7.44-7.34 (9H), 7.34-7.18 (17H), 7.07 (6H).

### Example 10

The melting point and the glass transition point of each of the arylamine compounds having a fluorenyl skeleton-containing heterocycle structure obtained in the above-described examples were measured using a high-sensitivity differential scanning calorimeter (DSC3100SA manufactured by Bruker AXS K.K.). Table 1 shows the results.

**Table 1**

| | Melting point | Glass transition point |
|---|---|---|
| Compound of Ex. 1 | - | 123°C |
| Compound of Ex. 2 | - | 143°C |
| Compound of Ex. 3 | 232°C | 132°C |
| Compound of Ex. 4 | 267°C | 132°C |
| Compound of Ex. 5 | 298°C | 160°C |
| Compound of Ex. 6 | 305°C | 152°C |
| Compound of Ex. 7 | 340°C | 152°C |
| Compound of Ex. 8 | 265°C | 148°C |
| Compound of Ex. 9 | 271°C | 164°C |

It is seen from Table 1 that the arylamine compounds having a fluorenyl skeleton-containing heterocycle structure represented by the general formula (A) had a glass transition point of 120°C or more, which means that these compounds were stable in the form of a thin film.

### Example 11

A vapor-deposited film (thickness: 100 nm) of the arylamine compound having a fluorenyl skeleton-containing heterocycle structure in the above-described examples was formed on an ITO substrate, and the work function was measured using an ionization potential measuring system (PYS-202 manufactured by Sumitomo Heavy Industries, Ltd.). Table 2 shows the results.

**Table 2**

| | Work function |
|---|---|
| Compound of Ex. 1 | 5.78 eV |
| Compound of Ex. 2 | 5.76 eV |
| Compound of Ex. 3 | 5.86 eV |
| Compound of Ex. 4 | 5.73 eV |
| Compound of Ex. 5 | 5.72 eV |
| Compound of Ex. 6 | 5.74 eV |
| Compound of Ex. 7 | 5.98 eV |
| Compound of Ex. 8 | 5.78 eV |
| Compound of Ex. 9 | 5.76 eV |

It is seen from Table 2 that the arylamine compounds having a fluorenyl skeleton-containing heterocycle structure represented by the general formula (A) had a work function higher than common hole-transporting materials such as NPD and TPD, which have a work function of 5.4 eV, and that these arylamine compounds thus had a better energy level. This means that the arylamine compounds having a fluorenyl skeleton-containing heterocycle structure have good hole-transporting capability.

### Example 12

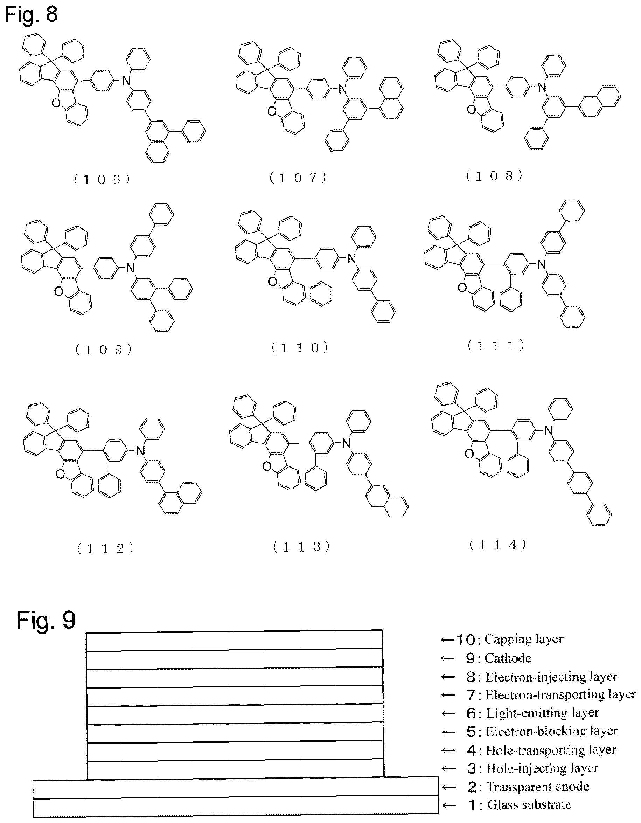

An organic EL device having the configuration as shown in Fig. 9 was prepared in the following manner: a reflective ITO electrode serving as a transparent anode 2 was formed on a glass substrate 1 beforehand, and a hole-injecting layer 3, a hole-transporting layer 4, an electron-blocking layer 5, a light-emitting layer 6, an electron-transporting layer 7, an electron-injecting layer 8, a cathode 9, and a capping layer 10 were vapor-deposited in this order on the ITO electrode.

Specifically, a glass substrate 1 on which an ITO film with a thickness of 50 nm, a reflective silver alloy film with a thickness of 100 nm, and an ITO film with a thickness of 5 nm were formed in this order was ultrasonically cleaned in isopropyl alcohol for 20 minutes, and then dried for 10 minutes on a hot plate heated to 250°C. After that, UV/ozone treatment was performed for 15 minutes. Then, the glass substrate with ITO was set inside a vacuum vapor deposition machine, and the pressure was reduced to 0.001 Pa or less.

Subsequently, an electron acceptor (Acceptor-1) having the structural formula below and a compound (HTM-1) having the structural formula below were vapor-deposited so as to coat the transparent anode 2 through binary vapor deposition at vapor deposition rates such that the ratio of the vapor deposition rate of Acceptor-1 to the vapor deposition rate of the compound (HTM-1) was 3:97, to thereby form the hole-injecting layer 3 with a thickness of 10 nm.

On this hole-injecting layer 3, the hole-transporting layer 4 (thickness: 140 nm) made of the compound (HTM-1) having the structural formula below was formed.

On this hole-transporting layer 4, the electron-blocking layer 5 (thickness: 5 nm) made of Compound (12) obtained in Example 1 was formed.

A compound (EMD-1) having the structural formula below and a compound (EMH-1) having the structural formula below were vapor-deposited on this electron-blocking layer 5 through binary vapor deposition at vapor deposition rates such that the ratio of the vapor deposition rate of the compound (EMD-1) to the vapor deposition rate of EMH-1 was 5:95, to thereby form the light-emitting layer 6 with a thickness of 20 nm.

A compound (ETM-1) having the structural formula below and a compound (ETM-2) having the structural formula below were vapor-deposited on this light-emitting layer 6 through binary vapor deposition at vapor deposition rates such that the ratio of the vapor deposition rate of the compound (ETM-1) to the vapor deposition rate of (ETM-2) was 50:50, to thereby form the electron-transporting layer 7 with a thickness of 30 nm.

On this electron-transporting layer 7, the electron-injecting layer 8 (thickness: 1 nm) made of lithium fluoride was formed.

On this electron-injecting layer 8, the cathode 9 (thickness: 12 nm) made of a magnesium-silver alloy was formed.

Finally, the capping layer 10 (thickness: 60 nm) made of a compound (CPL-1) having the structure below was formed.

Table 3 collectively shows the measurement results of light emission characteristics when a DC voltage was applied in the atmosphere at normal temperature to the organic EL device prepared in Example 12 above.

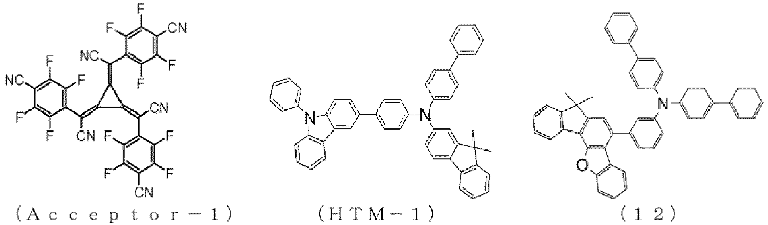

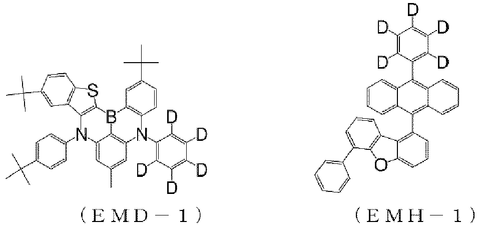

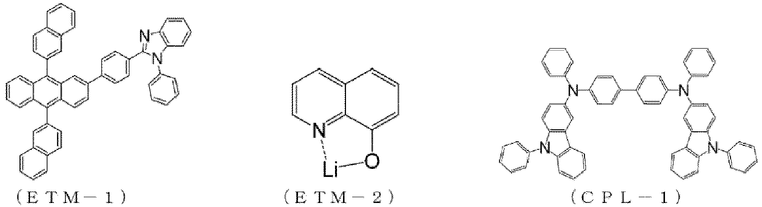

### Example 13

An organic EL device was prepared in the same manner as in Example 12, except that Compound (12) obtained in Example 1 used as the material for the electron-blocking layer 5 was replaced with Compound (14) obtained in Example 2. Table 3 collectively shows the measurement results of light emission characteristics when a DC voltage was applied in the atmosphere at normal temperature to the prepared organic EL device in the same measurement condition as in Example 12.

### Example 14

An organic EL device was prepared in the same manner as in Example 12, except that Compound (12) obtained in Example 1 used as the material for the electron-blocking layer 5 was replaced with Compound (52) obtained in Example 3. Table 3 collectively shows the measurement results of light emission characteristics when a DC voltage was applied in the atmosphere at normal temperature to the prepared organic EL device in the same measurement condition as in Example 12.

### Example 15

An organic EL device was prepared in the same manner as in Example 12, except that Compound (12) obtained in Example 1 used as the material for the electron-blocking layer 5 was replaced with Compound (7) obtained in Example 4. Table 3 collectively shows the measurement results of light emission characteristics when a DC voltage was applied in the atmosphere at normal temperature to the prepared organic EL device in the same measurement condition as in Example 12.

### Example 16

An organic EL device was prepared in the same manner as in Example 12, except that Compound (12) obtained in Example 1 used as the material for the electron-blocking layer 5 was replaced with Compound (24) obtained in Example 5. Table 3 collectively shows the measurement results of light emission characteristics when a DC voltage was applied in the atmosphere at normal temperature to the prepared organic EL device in the same measurement condition as in Example 12.

### Example 17

An organic EL device was prepared in the same manner as in Example 12, except that Compound (12) obtained in Example 1 used as the material for the electron-blocking layer 5 was replaced with Compound (26) obtained in Example 6. Table 3 collectively shows the measurement results of light emission characteristics when a DC voltage was applied in the atmosphere at normal temperature to the prepared organic EL device in the same measurement condition as in Example 12.

### Example 18

An organic EL device was prepared in the same manner as in Example 12, except that Compound (12) obtained in Example 1 used as the material for the electron-blocking layer 5 was replaced with Compound (43) obtained in Example 7. Table 3 collectively shows the measurement results of light emission characteristics when a DC voltage was applied in the atmosphere at normal temperature to the prepared organic EL device in the same measurement condition as in Example 12.

### Example 19

An organic EL device was prepared in the same manner as in Example 12, except that Compound (12) obtained in Example 1 used as the material for the electron-blocking layer 5 was replaced with Compound (61) obtained in Example 8. Table 3 collectively shows the measurement results of light emission characteristics when a DC voltage was applied in the atmosphere at normal temperature to the prepared organic EL device in the same measurement condition as in Example 12.

### Example 20

An organic EL device was prepared in the same manner as in Example 12, except that Compound (12) obtained in Example 1 used as the material for the electron-blocking layer 5 was replaced with Compound (94) obtained in Example 9. Table 3 collectively shows the measurement results of light emission characteristics when a DC voltage was applied in the atmosphere at normal temperature to the prepared organic EL device in the same measurement condition as in Example 12.

### Comparative Example 1

For comparison, an organic EL device was prepared in the same manner as in Example 12, except that Compound (12) obtained in Example 1 used as the material for the electron-blocking layer 5 was replaced with a compound (HTM-2) having the structural formula below (see Patent Literature 5, for example). Table 3 collectively shows the measurement results of light emission characteristics when a DC voltage was applied in the atmosphere at normal temperature to the prepared organic EL device in the same measurement condition as in Example 12.

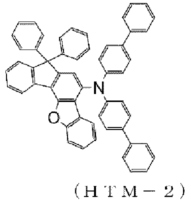

### Comparative Example 2

For comparison, an organic EL device was prepared in the same manner as in Example 12, except that Compound (12) obtained in Example 1 used as the material for the electron-blocking layer 5 was replaced with a compound (HTM-3) having the structural formula below (see Patent Literature 5, for example). Table 3 collectively shows the measurement results of light emission characteristics when a DC voltage was applied in the atmosphere at normal temperature to the prepared organic EL device in the same measurement condition as in Example 12.

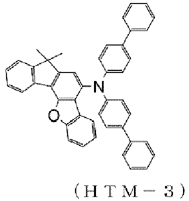

The device lifespan of each of the organic EL devices prepared in Examples and Comparative Examples was measured. The device lifespan was defined as follows: the organic EL device was driven by constant current to emit light at an initial luminance (the luminance when light emission started) of 1000 cd/m², and the time taken for the luminance to decay to 950 cd/m² (corresponding to 95% based on the initial luminance (100%): 95% decay) was determined and used as the device lifespan.

**Table 3**

| | Electron-blocking layer | (@10 mA/cm²) | | | | Device lifespan 95% decay |
|---|---|---|---|---|---|---|
| | | Voltage [V] | Luminance [cd/m²] | Luminous efficacy [cd/A] | Power efficiency [lm/W] | |
| Ex. 12 | Compound 12 | 3.42 | 1055 | 10.56 | 9.31 | 387 hrs. |
| Ex. 13 | Compound 14 | 3.38 | 1026 | 10.30 | 9.07 | 420 hrs. |
| Ex. 14 | Compound 52 | 3.41 | 958 | 9.58 | 8.44 | 349 hrs. |
| Ex. 15 | Compound 7 | 3.40 | 1014 | 10.14 | 8.52 | 791 hrs. |
| Ex. 16 | Compound 24 | 3.32 | 1125 | 11.27 | 10.14 | 633 hrs. |
| Ex. 17 | Compound 26 | 3.42 | 957 | 9.57 | 8.81 | 712 hrs. |
| Ex. 18 | Compound 43 | 3.48 | 851 | 8.52 | 7.50 | 410 hrs. |
| Ex. 19 | Compound 61 | 3.44 | 1108 | 11.11 | 9.84 | 407 hrs. |
| Ex. 20 | Compound 94 | 3.41 | 926 | 9.27 | 8.13 | 376 hrs. |
| Com. Ex. 1 | HTM-2 | 3.52 | 828 | 8.28 | 7.29 | 327 hrs. |
| Com. Ex. 2 | HTM-3 | 3.50 | 798 | 7.98 | 7.03 | 298 hrs. |

The following can be seen from Table 3. When a current with a current density of 10 mA/cm² was applied, the organic EL devices of Comparative Examples 1 and 2 had a luminous efficacy of 7.98 to 8.28 cd/A, and the organic EL devices of Example 12 to 20 had a luminous efficacy of 8.52 to 11.27 cd/A, which is higher efficacy. Moreover, while the organic EL devices of Comparative Examples 1 and 2 had power efficiencies of 7.03 to 7.29 lm/W, the organic EL devices of Example 12 to 20 had power efficiencies of 7.50 to 10.14 lm/W and therefore exhibited higher efficiency. Furthermore, the organic EL devices of Comparative Examples 1 and 2 had a device lifespan (95% decay) of 298 to 327 hours; in contrast, the organic EL devices of Example 12 to 20 had a device lifespan of 349 to 791 hours, that is known to be prolonged.

As is clear from the results described above, the organic EL devices each including the arylamine compound having a fluorenyl skeleton-containing heterocycle structure of the present invention, which has high hole mobility and excellent electron blockability, have higher luminous efficacy and a longer lifespan than conventional organic EL devices.

### Industrial Applicability

The organic EL device including the arylamine compound having a fluorenyl skeleton-containing heterocycle structure of the present invention has increased luminous efficacy and improved durability, and therefore, can be applied to a wide variety of uses such as home electric appliances and lighting equipment.

Moreover, the arylamine compound having a fluorenyl skeleton-containing heterocycle structure of the present invention can be applied not only to organic EL devices but also to electronic apparatuses in the fields of electrophotographic photoreceptors, image sensors, photoelectric transducers, solar cells, and the like.

### List of Reference Numerals

- 1: Glass substrate
- 2: Transparent anode
- 3: Hole-injecting layer
- 4: Hole-transporting layer
- 5: Electron-blocking layer
- 6: Light-emitting layer
- 7: Electron-transporting layer
- 8: Electron-injecting layer
- 9: Cathode
- 10: Capping layer

## Claims

1. An arylamine compound having a fluorenyl skeleton-containing heterocycle structure represented by a general formula (A) below: 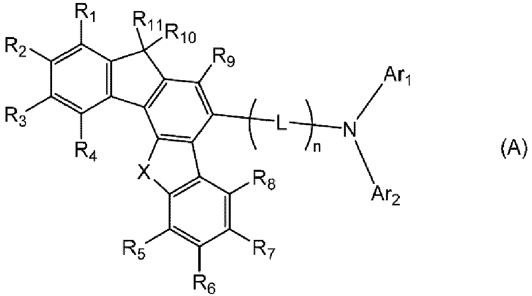
where R₁ to R₁₁ is the same or different, and each represent a hydrogen atom, a deuterium atom, a fluorine atom, a chlorine atom, a cyano group, a nitro group, a linear or branched alkyl group having 1 to 6 carbon atoms and optionally having a substituent, a cycloalkyl group having 5 to 10 carbon atoms and optionally having a substituent, a linear or branched alkenyl group having 2 to 6 carbon atoms and optionally having a substituent, a linear or branched alkyloxy group having 1 to 6 carbon atoms and optionally having a substituent, a cycloalkyloxy group having 5 to 10 carbon atoms and optionally having a substituent, a substituted or unsubstituted aromatic hydrocarbon group, a substituted or unsubstituted aromatic heterocyclic group, a substituted or unsubstituted fused polycyclic aromatic group, or a substituted or unsubstituted aryloxy group;
L represents a divalent group of a substituted or unsubstituted aromatic hydrocarbon group, a substituted or unsubstituted aromatic heterocyclic group, or a substituted or unsubstituted fused polycyclic aromatic group;
n is 1 or 2, and, when n is 2, L is the same or different;
An and Ar₂ is the same or different, and each represent a substituted or unsubstituted aromatic hydrocarbon group, a substituted or unsubstituted aromatic heterocyclic group, or a substituted or unsubstituted fused polycyclic aromatic group;
X represents an oxygen atom, a sulfur atom, or a nitrogen atom having a substituent; and
L and Ar₁ are optionally bonded to each other to form a ring via a single bond or a linking group selected from a substituted or unsubstituted methylene group, an oxygen atom, a sulfur atom, and a nitrogen atom having a substituent; and the same holds true for Ar₁ and Ar₂.

2. The arylamine compound having a fluorenyl skeleton-containing heterocycle structure as set forth in claim 1, wherein R₁₀ and R₁₁ are each a substituted or unsubstituted methyl group or a substituted or unsubstituted phenyl group.

3. The arylamine compound having a fluorenyl skeleton-containing heterocycle structure as set forth in claim 1 or 2, wherein X is an oxygen atom.

4. The arylamine compound having a fluorenyl skeleton-containing heterocycle structure as set forth in any one of claims 1 to 3, wherein L is a substituted or unsubstituted phenylene group, a substituted or unsubstituted biphenylene group, or a substituted or unsubstituted naphthylene group.

5. The arylamine compound having a fluorenyl skeleton-containing heterocycle structure as set forth in any one of claims 1 to 4, wherein n is 1.

6. An organic electroluminescence device comprising a pair of electrodes and one or more organic layers sandwiched therebetween, wherein at least one of the organic layers contains the arylamine compound having a fluorenyl skeleton-containing heterocycle structure as set forth in any one of claims 1 to 5.

7. The organic electroluminescence device as set forth in claim 6, wherein the organic layer is an electron-blocking layer.

8. The organic electroluminescence device as set forth in claim 6, wherein the organic layer is a hole-transporting layer.

9. The organic electroluminescence device as set forth in claim 6, wherein the organic layer is a hole-injecting layer.

10. The organic electroluminescence device as set forth in claim 6, wherein the organic layer is a light-emitting layer.

11. An electronic apparatus comprising an electronic component having a pair of electrodes and one or more organic layers sandwiched therebetween, wherein at least one of the organic layers contains the arylamine compound having a fluorenyl skeleton-containing heterocycle structure as set forth in any one of claims 1 to 5.
